# FASCICULE DE BREVET EUROPEEN

(11) **EP 2 614 046 B1**
(45) Date de publication et mention de la délivrance du brevet: **20.08.2014**
(21) Numéro de dépôt: 11773035.8
(22) Date de dépôt: 06.09.2011
(51) Int. Cl.: C07C 243/32, C07C 243/34, C07C 281/16, C07D 249/18, A61K 8/43, A61Q 19/08

(54) **NOUVEAUX DÉRIVÉS GUANIDINES EN SÉRIE CINNAMIQUE**
NEUARTIGE GUANIDINDERIVATE IN DER ZIMTREIHE
NOVEL GUANIDINE DERIVATIVES IN THE CINNAMIC SERIES

(30) Priorité: 07.09.2010 FR 1057087
(43) Date de publication de la demande: 17.07.2013
(73) Titulaire: Produits Chimiques Auxiliaires et de Synthese, 91160 Longjumeau (FR)
(72) Inventeur: LANCELOT, Jean Charles, F-14400 Tour En Bessin (FR); SUZANNE, Peggy, F-14120 Mondeville (FR); VOISIN-CHIRET, Anne-Sophie, F-14990 Bernieres-sur-mer (FR); PECQUET, Régis, F-91540 Mennecy (FR); JOSEPH, Jean-Christophe, F-91390 Morsang Sur Orge (FR); RAULT, Sylvain, F-14370 Moult (FR)
(74) Mandataire: Zimmermann, Alain
(86) Numéro de dépôt international: PCT/FR2011/052035
(87) Numéro de publication internationale: WO 2012/032257

(56) Documents cités:
- EP-A2- 0 339 496
- US-A- 5 130 324
- US-A- 5 476 849
- BUSEV A I ET AL: "Extraction of the complex of pentavalent molybdenum with thioglycolic acid in the presence of guanidine derivatives", JOURNAL OF ANALYTICAL CHEMISTRY OF THE USSR, CONSULTANTS BUREAU, NEW YORK, NY, US, vol. 20, 1 janvier 1965 (1965-01-01), pages 66-71, XP009146808, ISSN: 0021-8766
- WANG X ET AL: "Features and applications of reactions of alpha,beta-unsaturated N-acylbenzotriazoles with amino compounds", TETRAHEDRON, vol. 64, no. 27, 30 juin 2008 (2008-06-30) , pages 6510-6521, XP022695633, ELSEVIER SCIENCE PUBLISHERS, AMSTERDAM, NL ISSN: 0040-4020, DOI: 10.1016/J.TET.2008.04.054 [extrait le 2008-04-18] & WANG X ET AL: "Supplementary Data - Features and applications of reactions of alpha,beta-unsaturated N-acylbenzotriazoles with amino compounds", Sciencedirect Tetrahedron, Supplementary Data, vol. 64 30 juin 2008 (2008-06-30), pages S1-S51, XP002632618, Extrait de l'Internet: URL:http://www.sciencedirect.com/science/M iamiMultiMediaURL/B6THR-4S9P5KW-6/B6THR-4S 9P5KW-6-1/5289/html/S004040200800762X/3d6c fb358fa5ac1d9973df18ffddc401/f.doc [extrait le 2011-04-13]
- PARDIN C ET AL: "Cinnamoyl Inhibitors of Tissue Transglutaminase", JOURNAL OF ORGANIC CHEMISTRY, vol. 73, 27 juin 2008 (2008-06-27), pages 5766-5775, XP002632606,
- ALAN R KATRITZKY ET AL: "Preparation and Synthetic Applications of N-(alpha,Beta-Unsaturated Acyl)-Alpha-amino Acid Derivatives", HETEROCYCLES, ELSEVIER SCIENCE PUBLISHERS B.V. AMSTERDAM, NL, vol. 77, no. 2, 1 janvier 2009 (2009-01-01), pages 1249-1259, XP009146857, ISSN: 0385-5414
- TANAKA K ET AL: "SYNTHESES AND ANTI-INFLAMMATORYT AND ANALGESIC ACTIVITIES OF HYDROXAMIC ACIDS AND ACID HYDRAZIDES", CHEMICAL AND PHARMACEUTICAL BULLETIN, PHARMACEUTICAL SOCIETY OF JAPAN, TOKYO, JP, vol. 31, no. 8, 1 janvier 1983 (1983-01-01), pages 2810-2819, XP001026381, ISSN: 0009-2363
- CURTIUS TH ET AL: "Über das Hydrazid der m-Nitrozimtsäure und sein Verhalten gegen Salpetrigsäure", JOURNAL FUER PRAKTISCHE CHEMIE, vol. 107, 1 janvier 1924 (1924-01-01), pages 86-98, XP002632607, LEIPZIG
- FLAMMANG M ET AL: "Dérivés de l'acide caféique. II. Préparation de quelques amides et hydrazides de l'acide caféique. Etude pharmacologique des produits obtenus", CHIMICA THERAPEUTICA, SOCIETE D'ETUDES DE CHIMIE THERAPEUTIQUE, FR, vol. 4, 1 janvier 1969 (1969-01-01), pages 120-126, XP009146895,
- ANZAI K ET AL: "Studies on a new antibiotic, tuberin. V. Biological activities of tuberin analogues", JOURNAL OF ANTIBIOTICS, JAPAN ANTIBIOTICS RESEARCH ASSOCIATION, TOKYO, JP, vol. 15, 1 septembre 1962 (1962-09-01), pages 202-208, XP009146889, ISSN: 0021-8820
- ZHANG X ET AL: "A new procedure for preparation of carboxylic acid hydrazides", JOURNAL OF ORGANIC CHEMISTRY, vol. 67, 4 décembre 2002 (2002-12-04), pages 9471-9474, XP002632608,
- DATABASE REGISTRY CHEMICAL ABSTRACTS SERVICE, COLUMBUS, OHIO, US; 1 juillet 2007 (2007-07-01), XP002632609, Database accession no. RN: 940468-83-7 & DATABASE CHEMCATS CHEMICAL ABSTRACTS SERVICE, COLUMBUS, OHIO, US; XP002635369, Database accession no. 2044832181
- DATABASE REGISTRY CHEMICAL ABSTRACTS SERVICE, COLUMBUS, OHIO, US; 6 octobre 2003 (2003-10-06), XP002632610, Database accession no. RN: 599160-60-8 & DATABASE CHEMCATS CHEMICAL ABSTRACTS SERVICE, COLUMBUS, OHIO, US; XP002635370, Database accession no. 2081662740
- DATABASE REGISTRY CHEMICAL ABSTRACTS SERVICE, COLUMBUS, OHIO, US; 23 mai 2003 (2003-05-23), XP002632611, Database accession no. RN: 519151-15-6 & DATABASE CHEMCATS CHEMICAL ABSTRACTS SERVICE, COLUMBUS, OHIO, US; XP002635371, Database accession no. 0024062584
- DATABASE REGISTRY CHEMICAL ABSTRACTS SERVICE, COLUMBUS, OHIO, US; 29 juillet 2001 (2001-07-29), XP002632612, Database accession no. RN: 349428-82-6 & DATABASE CHEMCATS CHEMICAL ABSTRACTS SERVICE, COLUMBUS, OHIO, US; XP002635372, Database accession no. 2098520613
- DATABASE REGISTRY CHEMICAL ABSTRACTS SERVICE, COLUMBUS, OHIO, US; 19 février 2001 (2001-02-19), XP002632613, Database accession no. RN: 321979-09-3 & DATABASE CHEMCATS CHEMICAL ABSTRACTS SERVICE, COLUMBUS, OHIO, US; XP002635373, Database accession no. 2098481000
- DATABASE CA [Online] CHEMICAL ABSTRACTS SERVICE, COLUMBUS, OHIO, US; BARR, KENNETH J. ET AL: "Preparation of oxadiazoline antiproliferative agents", XP002632614, extrait de STN Database accession no. 2002:807579 & ZA 200 000 419 A (ABBOTT LAB) 28 juin 2000 (2000-06-28)
- DATABASE CA [Online] CHEMICAL ABSTRACTS SERVICE, COLUMBUS, OHIO, US; KATRITZKY, ALAN R. ET AL: "One-pot synthesis of cinnamoyl hydrazides", XP002632615, extrait de STN Database accession no. 2002:885932 & KATRITZKY A R ET AL: "One-pot synthesis of cinnamoyl hydrazides", ARKIVOC (GAINESVILLE, FL, UNITED STATES) [ONLINE COMPUTER FILE], vol. 9, 1 janvier 2001 (2001-01-01), pages 19-23,
- DATABASE REGISTRY CHEMICAL ABSTRACTS SERVICE, COLUMBUS, OHIO, US; 1 février 2009 (2009-02-01), XP002632616, Database accession no. RN: 1098351-36-0 & DATABASE CHEMCATS CHEMICAL ABSTRACTS SERVICE, COLUMBUS, OHIO, US; XP002635374, Database accession no. 2076000482
- DATABASE REGISTRY CHEMICAL ABSTRACTS SERVICE, COLUMBUS, OHIO, US; 1 février 2009 (2009-02-01), XP002632617, Database accession no. RN: 1098355-59-9 & DATABASE CHEMCATS CHEMICAL ABSTRACTS SERVICE, COLUMBUS, OHIO, US; XP002635375, Database accession no. 2075931472
- EDMONT D ET AL: "Synthesis and evaluation of quinoline carboxyguanidines as antidiabetic agents", BIOORGANIC & MEDICINAL CHEMISTRY LETTERS, PERGAMON, ELSEVIER SCIENCE, GB, vol. 10, no. 16, 21 août 2000 (2000-08-21) , pages 1831-1834, XP004216010, ISSN: 0960-894X, DOI: DOI:10.1016/S0960-894X(00)00354-1
- THORSTEN LIFKA ET AL: "3,6-Bis(2-arylethenyl)-1,2,4,5-tetrazine - Synthese, Fl�ssigkristallinit�t und Photochemie", JOURNAL F&#XFFFD;R PRAKTISCHE CHEMIE/CHEMIKER-ZEITUNG, vol. 337, no. 1, 1 janvier 1995 (1995-01-01), pages 641-646, XP55012270, ISSN: 0941-1216, DOI: 10.1002/prac.199533701137

## Description

La présente invention a pour objet de nouveaux dérivés guanidines en série cinnamique, leur procédé de préparation et leur utilisation pour la préparation de compositions aux propriétés anti-glycation, notamment en cosmétologie.

Le vieillissement de la peau est provoqué par trois principaux facteurs : les radicaux libres, les rayonnements UV et le processus de glycation.

La glycation est une réaction de liaison entre un sucre et une protéine qui conduit à la formation de produits de glycation qui ne sont ni détruits ni évacués des cellules et dont l'accumulation provoque un vieillissement des cellules.

L'aminoguanidine est un agent connu pour ses propriétés anti-glycation.

Toutefois, il existe un besoin constant de fournir des agents aux propriétés anti-glycation encore meilleures.

Le document US5476849 décrit des dérivés 4-(guanidinylamino-carbonyl)anilines possédant des propriétés anti-glycation (colonne 7, ligne 25 ; colonne 8, ligne 10 ; colonne 8, lignes 13-18) mais ces dérivés diffèrent, par la nature de l'acide fixé au groupe aminoguanidine, de ceux proposés ci-après par la Demanderesse dans la présente demande de brevet : les nouveaux dérivés proposés par la Demanderesse ont, de façon inattendue, des propriétés anti-glycation améliorées.

D'autres dérivés de l'amino-guanidine sont connus, dans l'art antérieur, comme potentiels inhibiteurs de glycation (voir notamment les documents US5130324 et EP339496) sans que cette propriété ait été effectivement établie.

La Demanderesse a donc maintenant conçu de nouveaux dérivés guanidines en série cinnamique, de structure originale, leur conférant des propriétés intéressantes de lutte contre le vieillissement de la peau, à savoir des propriétés apaisantes, associées à des propriétés anti-oxydantes et anti-glycation, de telles propriétés en faisant des principes actifs de choix dans le domaine cosmétologique.

Les nouveaux dérivés selon l'invention répondent plus précisément à la formule générale (I) : 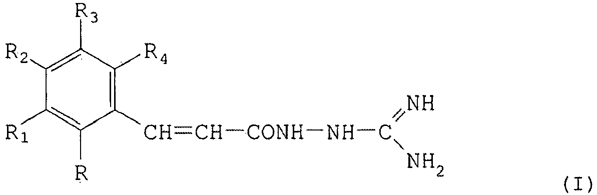 dans laquelle :
- R représente un atome d'hydrogène ou un groupe alkoxy en C1-C4,
- R1 représente un atome d'hydrogène, un groupe alkoxy en C1-C4, un groupe NO2 ou un groupe OH,
- R2 représente un atome d'hydrogène, un groupe alkoxy en C1-C4 ou un groupe OH,
- R1 et R2 peuvent également former ensemble un groupe OCH2O,
- R3 représente un atome d'hydrogène ou un groupe alkoxy en C1-C4,
- R, R1, R2 et R3 ne pouvant simultanément représenter un atome d'hydrogène, et
- R4 représente un atome d'hydrogène,
ainsi que leurs sels et leurs isomères.

Selon un mode de réalisation l'ensemble (R, R1, R2, R3, R4) est choisi dans le groupe constitué par (H, NO2, H, H, H), (alkoxy en C1-C4, H, H, H, H) et (H, alkoxy en C1-C4, OH, H, H).

Selon un autre mode de réalisation, R1 et R2 forment ensemble un groupe OCH2O, et R, R3 et R4 représentent chacun un atome d'hydrogène.

La présente invention s'étend à tous les isomères des dérivés de formule (I) et aux sels de ces derniers.

En particulier, la présente invention s'étend aux sels d'addition d'un acide minéral tel que HCl, HBr et H2SO4, ainsi qu'aux sels d'addition d'un acide organique tel que l'acide méthanesulfonique, l'acide benzoïque, l'acide salicylique, l'acide lactique, l'acide citrique, l'acide malique D ou L, l'acide glucuronique D et l'acide hyaluronique.

Les nouveaux dérivés, isomères et sels selon l'invention peuvent être préparés par un procédé qui comprend :
(i) la réaction du nitrate de 3,5-diméthylpyrazole-1-carboxamidine avec un composé de formule générale (III) suivante : 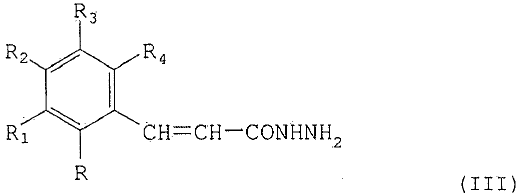 dans laquelle R, R1, R2, R3 et R4 ont la même signification que dans la formule (I),
   pour obtenir le nitrate d'un composé de formule (I),
(ii) l'alcalinisation du nitrate obtenu à l'étape (i), pour obtenir le composé de formule (I), et
(iii) la salification éventuelle du composé de formule (I) obtenu à l'étape (ii) par un acide minéral ou organique approprié.

Le composé de formule (III) intervenant dans l'étape (i) du procédé peut être obtenu par différentes voies.

Selon un mode de réalisation, le composé de formule (III) est préparé en faisant réagir de l'hydrazine avec un composé de formule générale (II) suivante (procédé A) : 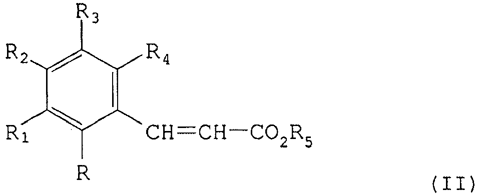 dans laquelle R, R1, R2, R3 et R4 ont la même signification que dans la formule (I) et R5 représente un groupe alkyle en C1-C4,

Alternativement, le composé (III) est préparé selon les étapes suivantes (procédé B) :
(iv) la réaction du 1-(méthylsulfonyl)benzotriazole avec un composé de formule générale (IV) suivante : 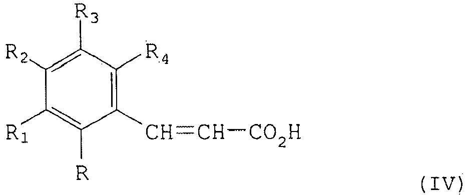 dans laquelle R, R1, R2, R3 et R4 ont la même signification que dans la formule (I),
   pour obtenir un composé de formule générale (V) suivante : 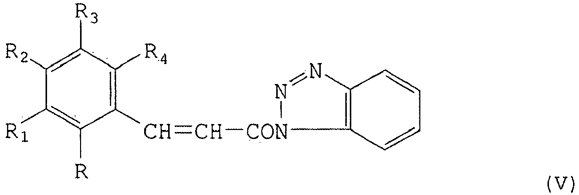 dans laquelle R, R1, R2, R3 et R4 ont la même signification que dans la formule (I), et
(v) la réaction du composé de formule (V) obtenu à l'étape (iv) avec de l'hydrazine pour obtenir le composé de formule (III).

Lorsque l'on utilise un composé de formule (IV) comportant un groupement -OH, il est préférable de protéger cette fonction, avant d'effectuer l'étape (iv).

L'invention s'étend aussi à l'utilisation des dérivés de formule (I), de leurs sels et isomères pour la préparation d'une composition anti-glycation, en particulier dans une composition cosmétique.

Enfin, l'invention concerne une composition cosmétique comprenant au moins un dérivé de formule (I), son isomère ou sel et un véhicule acceptable sur le plan cosmétique.

La préparation d'un certain nombre de dérivés de formule (I), d'intermédiaires de synthèse de formule (III) et d'intermédiaires de synthèse de formule (V), regroupés respectivement dans les tableaux 1, 2 et 3 va maintenant être décrite plus en détails.

**Tableau 1 Composés de formule (I)**

| | R | R1 | R2 | R3 | R4 |
|---|---|---|---|---|---|
| 29 | H | OCH3 | OH | H | H |
| 30 | H | OCH3 | OH | H | H |
| 31 | H | OCH3 | OH | H | H |
| 32 | H | OCH3 | OH | H | H |
| 33 | H | OCH3 | OH | H | H |
| 34 | H | OCH3 | OH | H | H |
| 35 | H | OCH3 | OH | H | H |
| 36 | H | OCH3 | OH | H | H |
| 38 | H | R1+ R2 forment un groupe OCH2O | | H | H |
| 39 | H | NO2 | H | H | H |
| 40 | OCH3 | H | H | H | H |
| 41 | H | OCH3 | H | H | H |
| 42 | H | OH | H | H | H |
| 43 | OC2H5 | H | H | H | H |
| 44 | OCH3 | H | H | OCH3 | H |
| 45 | OC2H5 | H | H | H | H |
| 46 | OCH3 | H | H | OCH3 | H |
| 47 | OC2H5 | H | H | H | H |
| 48 | OCH3 | H | H | OCH3 | H |

**Tableau 2 Composés de formule (III)**

| | R | R1 | R2 | R3 | R4 |
|---|---|---|---|---|---|
| 15 | H | R1+ R2 forment un groupe OCH2O | | H | H |
| 16 | H | NO2 | H | H | H |
| 17 | OCH3 | H | H | H | H |
| 18 | H | OCH3 | H | H | H |
| 19 | H | OH | H | H | H |
| 21 | H | H | OCH3 | H | H |
| 22 | OC2H5 | H | H | H | H |
| 23 | H | OCH3 | OCH3 | H | H |
| 24 | OCH3 | H | H | OCH3 | H |
| 25 | H | OH | OCH3 | H | H |
| 26 | H | OCH3 | OH | H | H |
| 27 | H | OCH3 | OCH3 | OCH3 | H |
| 28 | OCH3 | OCH3 | OCH3 | H | H |

**Tableau 3 Composés de formule (V)**

| | R | R1 | R2 | R3 | R4 |
|---|---|---|---|---|---|
| 2 | H | R1+ R2 forment un groupe OCH2O | | H | H |
| 3 | H | NO2 | H | H | H |
| 4 | OCH3 | H | H | H | H |
| 5 | H | OCH3 | H | H | H |
| 6* | H | OCOCH3 | H | H | H |
| 7 * | H | OCH3 | OCOCH3 | H | H |
| 8 | H | H | OCH3 | H | H |
| 9 | OC2H5 | H | H | H | H |
| 10 | OCH3 | H | H | OCH3 | H |
| 11* | H | OCOOCH3 | OCH3 | H | H |
| 12 | H | OCH3 | OCH3 | OCH3 | H |
| 13 | OCH3 | OCH3 | OCH3 | H | H |

| | | | | | |
|---|---|---|---|---|---|
| * composés non revendiqués | | | | | |

On donnera ci-après un certain nombre d'exemples illustrant la synthèse des intermédiaires de synthèse et des dérivés selon l'invention ; les numéros apparaissant dans ces exemples correspondent aux composés portant respectivement les mêmes numéros dans les Tableaux 1 à 3 ci-dessus.

### Préparation des intermédiaires de synthèse de formule (V)

### Exemple 1 (non revendiqué) : (E)-1-(benzotriazol-1-yl)-3-phényl-2-propèn-1-one

On agite à température ambiante dans 50ml de tétrahydrofurane 1,42 g (0,01 mole) d'acide trans cinnamique (composé de formule IV) et 1,97g (0,01 mole) de 1-(méthylsulfonyl)benzotriazole, puis on ajoute goutte à goutte 0,014 mole de triéthylamine. La solution est ensuite portée au reflux pendant 24 heures, puis évaporée sous pression réduite. Le résidu est repris dans 60 ml d'eau et extrait par 70 ml d'acétate d'éthyle. La phase organique est séchée, filtrée puis concentrée sous vide. La poudre obtenue est reprise dans 50 ml d'acétonitrile et essorée.

On obtient 2,24g d'une poudre blanche.
Point de fusion : 157°C

### Exemple 2 : 1-[(2E)-3-(1,3-benzodioxol-5-yl)prop-2-ènoyl]-1H-1,2,3benzotriazole

On utilise le mode opératoire de l'exemple 1 à partir de 1,92 g (0,01 mole) d'acide 3,4-(méthylènedioxy)cinnamique (composé de formule IV), de 1,97 g (0,01 mole) de 1-(méthylsulfonyl)benzotriazole et de triéthylamine.

On obtient 1.80g d'une poudre jaune.
Point de fusion : 198 °C
Spectre IR (Kbr) : 1693 (C=O)
Spectre RMN (CDCl3) : 6,00 (s, 2H, CH2), 6,22 (d, J2-3 : 15,60 Hz, 1H, H2), 6,81, 6,98 (m, 2H, H5',6'), 7,48 (m, 5H, H-benzotriazole, H2'), 7,51 (d, J3-2 : 15,60 Hz, 1H, H3)

### Exemple 3 : (E)-1-(benzotriazol-1-yl)-3-(3-nitrophényl)-2-propèn-1-one

On utilise le mode opératoire de l'exemple 1 à partir de 1,93 g d'acide 3-nitro cinnamique (composé de formule IV) et de 1,97g de 1-(méthylsulfonyl)benzotriazole et de 0,014 mole de triéthylamine.

On obtient 2,68g d'une poudre blanche
Point de fusion : 230°C
Spectre IR (KBr) : 1704 (C=O)
Spectre RMN 1H (DMSO d6) : 7,65 (d, d, J : 8,1,7,5 Hz , 1H), 7,78 (dd, 2H), 8,25, 8,27, 8,31 (m, 6H), 8,68 (s, 1H, H2').

### Exemple 4 : (E)-1-(benzotriazol-1-yl)-3-(2-méthoxyphényl)-2-propèn-1-one

On utilise le mode opératoire de l'exemple 1, à partir de 1,78 g (0,01 mole) d'acide 2-méthoxycinnamique (composé de formule IV), de 1,97g (0,01 mole) de 1-(méthylsulfonyl)benzotriazole et de 0,014 mole de triéthylamine.

On obtient 2,30g d'une poudre blanche
Point de fusion : 158°C
Spectre IR (KBr) : 1699 (C=O)
Spectre RMN 1H (CDC13) : 3,97 (s, 3H, OCH3), 6,99, 7,02 (multiplet, 2H), 7,69, 7,75 (multiplet, 2H), 8,13, (d, J3'4': 8,75 Hz, 1H, H3'=, 8,21 (d, J2-3 : 15,80 Hz, 1H, H2), 8,44 (d, J: 7,79 Hz, 1H, H benzotriazole), 8,51 (d, J3-2: 15,60 Hz, 1H, H3).

### Exemple 5: (E)-1-(benzotriazol-1-yl)-3-(3-méthoxyphényl)-2-propèn-1-one

On utilise le mode opératoire de l'exemple 1 à partir de 1,78 g (0,01 mole) d'acide 3-méthoxycinnamique (composé de formule IV), de 1,97 g (0,01 mole) de 1-(méthylsulfonyl)benzotriazole et de 0,014 mole de triéthylamine.

On obtient 2,15g d'une poudre blanche
Point de fusion : 156°C
Spectre IR (KBr) : 1710 (C=O)
Spectre RMN 1H (CDCl3) : 3,90 (s, 3H, OCH3)), 7,05 (d, 1H, H benzotriazole), 7,25 (d, 1H, H benzotriazole), 7,37 (m, 2H, H5', H benzotriazole), 7,53 (d, d, J5'-6', 5-'4' : 7,90, 7,30 Hz, 1H, H5'), 8,11 (m, 2H, H2', H benzotriazole), 8,14 (d, J2-3 : 16,1 Hz, 1H, H1), 8,42 (d, J3-2 : 16,1 Hz, 1H, H3).

### Exemple 6 (non revendiqué) : (E)-1-(benzotriazol-1-yl)3-3(3-acétyloxyphényl)-2-propèn-1-one

On utilise le mode opératoire de l'exemple 1 à partir de 2,05 g (0,01 mole) d'acide (2E)-3-(3-acétyloxyphényl)acrylique, de 1,97g (0,01 mole) de 1-méthylsulfonyl)benzotriazole et de 0,014 mole de triéthylamine.

On obtient 2,1 g d'une poudre blanche
Point de fusion : 156°C
Spectre IR (KBr) : 1750, 1690 (C=O)
Spectre RMN 1H (DMSO d6) : 2,26 (s, 3H, OCOCH3), 7,10 (d, 1H, H benzotriazole), 7,23 (d, 1H, H benzotriazole), 7,30 (m, 2H, H6', H benzotriazole), 7,50 (d, d J5'-6' : 7,85 Hz, J5'-4' : 7,30 Hz, 1H, H5'), 8,02 (m, 2H, H benzotriazole), 8,10 (d, J2-3 : 15,8 Hz 1H, H2), 8,36 (d, J3-2 : 15,8 Hz, 1H, H3).

Le composé de l'exemple 6 peut être préparé à partir de l'acide 3-hydroxycinnamique (composé de formule IV), dont on a préalablement protégé la fonction hydroxy de la manière suivante.

On agite dans 50 ml de pyridine pendant 24 heures 5 g (0,030 mole) d'acide 3-hydroxycinnamique ne présence de 3 ml d'anhydride acétique et de 0,16 g (0,013 mole) de diméthylaminopyridine. La solution est concentrée sous pression réduite, le résidu est repris par 150 ml d'une solution à 10% d'acide chlorhydrique et extraite par 2 fois 120 ml d'acétate d'éthyle.

On obtient l'acide (2E)-3-(3-acétyloxyphényl)acrylique qui pourra être utilisé pour la préparation du composé de l'exemple 6.

### Exemple 7 (non revendiqué) : (E)-1-(benzotriazol-1-yl)-3-(4-acétyloxy-3-méthoxyphényl)-2-propèn-1-one

On utilise le mode opératoire de l'exemple 1 à partir de 2,36 g (0,01 mole) d'acide 4-(acétyloxy)férulique, de 1,97 g (0,01 mole) de 1-(méthylsulfonyl)benzotriazole et de 0,014 mole de triéthylamine.

On obtient 3,01g d'une poudre blanche.
Point de fusion : 160°C
Spectre IR (KBr) : 1752, 1690 (C=0)
Spectre RMN 1H (DMSO d6) : 2,27 (s,3H, COCH3), 3,88 (s, 3H, OCH3), 7,22 (d, J: 7,83 Hz, 1H, H benzotriazole), 7,50 (d, J6'-5' : 8,30 Hz, 1H, H6'), 7,2 (t, 1H, H benzotriazole), 7,70 (s, 1H, H2'), 7,79 (t, 1H, H benzotriazole), 8,10 (m, 2H, H2, H3), 8,27 (d, J : 7,38 Hz, 1H, H benzotriazole), 8,33 (d, J5'-6' : 7,83 Hz, 1H, H5')

Le composé de l'exemple 7 peut également être obtenu à partir d'un composé de formule IV, à savoir l'acide férulique dont on a préalablement protégé la fonction hydroxy de la manière suivante.

A température ambiante, on met 50 g (0,258 mole) d'acide férulique en solution dans de la pyridine. On ajoute 1,6g (0,0131 mole) de DMAP au milieu réactionnel, puis on ajoute goutte à goutte 27 ml d'anhydre acétique. Après 18 heures d'agitation à température ambiante, on verse la solution dans 1 litre d'eau froide et le précipité formé est essoré, lavé 3 fois avec 500 ml d'eau, séché et recristallisé dans l'éthanol.

On obtient l'acide 4-(acétyloxy)férulique, qui pourra être utilisé pour la préparation du composé de l'exemple 7.

### Exemple 8 : (E)-1-(benzotriazol-1-yl)-3-(4-méthoxyphényl)-2-propèn-1-one

### On utilise le mode opératoire de l'exemple 1 à partir de 1,78 g (0,01 mole) d'acide 4-méthoxycinnamique (composé de formule IV), de 1,97 g (0,01 mole) de 1-(méthylsulfonyl)benzotriazole et de 0,014 mole de triéthylamine.

On obtient 2,1g d'une poudre blanche.
Point de fusion : 186°C
Spectre IR (KBr) : 1697 (C=O)
Spectre RMN 1H (CDCl3) : 3,88 (s, 3H, OCH3), 6,62 (d, J2-3 : 16,50 Hz, 1H), 6,99 (d, 2H), 7,46 (m, 3H), 7,48 (d, 2H), 8,02 (d, J : 6,83 Hz, 1H), 8,09 (d, J : 7,83 Hz, 1H)

### Exemple 9 : (E)-1-(benzotriazol-1-yl)-3-(2-éthoxyphényl)-2-propèn-1-one

On utilise le mode opératoire de l'exemple 1 à partir de 1,92 g (0,01 mole) d'acide 2-éthoxycinnamique (composé de formule IV), de 1,97 g (0,01 mole) de 1-(méthylsulfonyl)benzotriazole et de 0,014 mole de triéthylamine.

On obtient 2 g d'une poudre blanche.
Point de fusion : 150°C
Spectre IR (KBr) : 1711 (C=O)
Spectre RMN 1H (CDCl3) : 1,57 (t, 3H, CH3), 4,19 (q, 2H, CH2), 6,97, 7,02 (multiplet, 2H), 7,41, 7,52 (multiplet, 2H), 7,67, 7,73 (multiplet, 2H), 8,13 (d, J3'-4' : 8,79 Hz, 1H, 3H'), 8,25 (d, J2-3 : 16,58 Hz, 1H, H2), 8,44 (d, J : 8,80 Hz, 1H, H benzotriazole), 8,50 (d, J3-2 : 15,60 Hz, 1H, H3).

### Exemple 10 : (E)-1-(benzotriazol-1-yl)-3-(2,5-diméthoxyphényl)-2-propèn-1-one

On utilise le mode opératoire de l'exemple 1 à partir de 2,08 g (0,01 mole) d'acide 2,5-diméthoxycinnamique (composé de formule IV), de 1,97 g (0,01 mole) de 1-(méthylsulfonyl)benzotriazole et de 0,014 mole de triéthylamine.

On obtient 1,98 g d'une poudre blanche.
Point de fusion : 144°C
Spectre IR (KBr) : 1700 (C=O)
Spectre RMN 1H (CDCl3) : 3,85 (s, 3H, OCH3), 3,92 (s, 3H, OCH3), 6,92 (d, 1H, H benzotriazole), 7,03 (q, J6'-4' : 2,91 Hz, 1H, H6'), 7,54, 7,66 (multiplet, 2H, H benzotriazole), 8,17 (q, J2-3 : 15,60 Hz, J3'-4' : 8,79 Hz, 2H, H2, H3'), 8,24 (d, J : 7,83 Hz, 1H, H benzotriazole), 8,50 (d, J3-2 : 15,60 Hz, 1H, H3)

### Exemple 11 (non revendiqué) (E)-1-(benzotriazol-1-yl)-3-(3-acétyloxy-4-méthoxyphényl)-2-propèn-1-one

On utilise le mode opératoire de l'exemple 1 à partir de 2,36 g (0,01 mole) d'acide (2E)-3-[(3-acétyloxy)-4-méthoxyphényl] acrylique, de 1,97 g (0,01 mole) de 1-(méthylsulfonyl)benzotriazole et de 0,014 mole de triéthylamine.

On obtient 2,95 g d'une poudre blanche.
Point de fusion : 152°C
Spectre IR (KBr) : 1754, 1693 (C=O)
Spectre RMN 1H (DMSO d6) : 2,28 (s,3H, COCH3), 3,92 (s, 3H, OCH3), 7,24 (d, J: 8,79 Hz, 1H, H benzotriazole), 7,60, 7,79 (m, 4H, H5', H benzotriazole), 7,94 (d, J2-3 : 15,60 Hz, 1H, H2), 8,09 (d, J3-2 : 15,62 Hz, 1H, H3), 8,26 (d, J5'-6': 7,79 Hz, 1H, H5'), 8,31 (d, J6'-5' : 7,80 Hz, 1H, H6')

Le composé acide (2E)-3-[(3-acétyloxy)-4-méthoxyphényl]acrylique, peut être obtenu à partir d'un composé de formule IV, à savoir l'acide 3-hydroxy-4-méthoxycinnamique de la manière suivante.

On agite dans 50 ml de pyridine pendant 24 heures, 15 g (0,077 mole) d'acide 3-hydroxy-4-méthoxycinnamique en présence de 8,1 ml d'anhydride acétique et de 0,48 g (0,0039 mole) de diméthylaminopyridine. La solution est concentrée sous pression réduite, le résidu est repris par 150 ml d'une solution à 10 % d'acide chlorhydrique et extraite par 2 fois dans 120 ml d'acétate d'éthyle.

Exemple 12 : (E)-1-(benzotriazol-1-yl)-3-(3,4,5-triméthoxyphényl)-2-propèn-1-one

On utilise le mode opératoire de l'exemple 1 à partir de 2,38 g (0,01 mole) d'acide 3,4,5-triméthoxycinnamique (composé de formule IV), de 1,97 g (0,01 mole) de 1-(méthylsulfonyl)benzotriazole et de 0,014 mole de triéthylamine.

On obtient 3 g d'une poudre jaune.
Point de fusion : 136°C

### Exemple 13 : (E)-1-(benzotriazol-1-yl)-3-(2,3,4-triméthoxyphényl)-2-propèn-1-one

On utilise le mode opératoire de l'exemple 1 à partir de 1,97 g (0,01 mole) d'acide 2,3,4,-triméthoxycinnamique (composé de formule IV), de 1,97 g (0,01 mole) de 1-(méthylsulfonyl)benzotriazole et de 0,014 mole de triéthylamine.

On obtient 3,1 g d'une poudre blanche.
Point de fusion : 138°C
Spectre IR (KBr) : 1706 (C=O)
Spectre RMN 1H (DMSO d6) : 3,78 (s, 3H, OCH3), 3,87 (s, 3H, OCH3), 3,91 (s, 3H, OCH3), 6,95 (d, J: 8,75 Hz, 1H, H benzotriazole), 7,15 (m, 3H, H benzotriazole), 8,02 (d, J3-2 : 16,58 Hz, 1H, H2), 8,20 (d, J3-2 : 16,6 Hz, 1H, H3), 8,32 (d, J5'-6' : 7,83 Hz, 1H, H5')

### Préparation des intermédiaires de synthèse de formule (III)

### Exemple 14 (non revendiqué) : (2E)-3-phénylacrylohydrazide

Cet exemple illustre, pour un composé du même type que les composés de formule (III) selon l'invention mais dans lequel chacun de R, R1, R2, R3 et R4 représente un atome d'hydrogène, les procédés de préparation A et B qui peuvent être employés.

### Procédé A

On agite à 70°C pendant 1h30 5 g (0,031 mole) de cinnamate de méthyle (composé de formule II) dans 50 ml d'hydrazine hydratée.

Après refroidissement, la solution est concentrée sous vide, le résidu est agité pendant une heure dans 60 ml d'eau froide. Le précipité est essoré, lavé à l'acétonitrile puis à l'éther et séché.

On obtient 0,30g d'une poudre blanche.
Point de fusion : 116 °C
Spectre IR (KBr) : 3234 (NH), 1650 (C=O)
Spectre RMN 1H (DMSO) : 3,60 (s, 2H, NH2), 6,38 (d, J2.3 : 15,7Hz, 1H, H2), 7,00 (s, 1H, NH), 7,38, 7,52 (m, 5H, H phényl), 7,71 (d, J3.2: 15,7 Hz, 1H, H3)

### Procédé B

On agite à température ambiante dans 40 ml de tétrahydrofurane 3 g (0, 0120 mole) du dérivé (E)-1-(benzotriazol-1-yl-3-phényl-2-propen-1one) (composé de formule V, obtenu dans l'exemple 1) en présence de 0,66 g (0,0132 mole) d'hydrate d'hydrazine. Après 3 heures d'agitation, la solution est concentrée sous pression réduite, le résidu est repris par 50 ml d'une solution de carbonate de potassium et extraite par 100 ml d'acétate d'éthyle. La phase organique est séchée sur sulfate de magnésium, puis concentrée sous vide.

On obtient 1,65g d'une poudre blanche.
Point de fusion : 116°C

Les spectres IR et RMN 1H sont identiques à ceux obtenus selon le procédé A.

### Exemple 15 : (2E)-(3,4-méthylènedioxy)acrylohydrazide

On utilise le même mode opératoire que le procédé B de l'exemple 14, à partir de 2,93 g (0,01 mole) du N-acylbenzotriazole (composé de formule V) obtenu dans l'exemple 2 et de 0,6 g (0,012 mole) d'hydrazine hydratée.

On obtient 1,3g d'une poudre blanche.
Point de fusion : 136°C
Spectre IR (KBr) : 3264 (NH-NH2), 1656 (C=O)
Spectre RMN 1H (CDCl3) : 4,19 (s, 2H, NH2), 6,00 (s, 2H, OCH2O), 6,22 (d, J2-3: 15,60 Hz, 1H, H2), 6,81 (d, J5'-6': 7,73 Hz, 1H, H5'), 7,05 (m, 2H, H2', H6'), 7,14 (s, 1H, NH), 7,61 (d, J3-2 : 15,65 Hz, 1H, H3).

### Exemple 16 : (2E)-3-(3-nitrophényl)acrylohydrazide

On utilise le même mode opératoire que le procédé B de l'exemple 14, à partir de 2,94 g (0,01 mole) de N-acylbenzotriazole (composé de formule V) obtenu dans l'exemple 3, et de 0,6 g (0, 012 mole) d'hydrazine hydratée.

On obtient 1,80 g d'une poudre blanche
Point de fusion : 196°C
Spectre IR (KBr) : 3332, 3237 (NH-NH2), 1610 (C=O)
Spectre RMN 1H (DMSO d6) : 4,51 (s, 2H, NH2), 6,74 (d, J2-3: 15,58 Hz, 1H, H3), 7,71 (t, 1H, H5'), 8, 10 (d, J6'-5': 7,79 Hz, 1H, H6'), 8,20 (d, J4'-5' : 7,80 Hz, 1H, H4').8,37 (s, 1H, H2'), 9,42 (s, 1H, NH).

### Exemple 17 : (2E)-3-(2-méthoxyphényl)acrylohydrazide

On utilise le même mode opératoire que le procédé B de l'exemple 14, à partir de 2,79 g (0,01 mole) de N-acylbenzotriazole (composé de formule V) obtenu dans l'exemple 4, et de 0,6 g (0, 012 mole) d'hydrazine hydratée.

On obtient 1,45 g d'une poudre blanche
Point de fusion : 120°C
Spectre IR (KBr) : 3306, 3300 (NH-NH2), 1603 (C=O)
Spectre RMN 1H (CDCl3) : 3,85 (s, 3H, OCH3), 4,11 (s, 2H, NH2), 6,54 (s, 2H, (d, J2-3: 15,60 Hz, 1H, H2), 6,88 (multiplet, 2H, H5'-6'), 7,31 (d, d, 1H', H4'), 7,44 (d, J3'-4' : 8,79 Hz, 1H, H3'), 7,50 (s, 1H, NH), 7,95 (d, J3-2 : 15,58 Hz, 1H, H3).

### Exemple 18 : (2E)-3-(3-méthoxyphényl)acrylohydrazide

On utilise le même mode opératoire que le procédé B de l'exemple 14, à partir de 2,79 g (0,01 mole) de N-acylbenzotriazole (composé de formule V) obtenu dans l'exemple 5, et de 0,6 g (0, 012 mole) d'hydrazine hydratée.

On obtient 1,5 g d'une poudre blanche
Point de fusion : 130°C
Spectre IR (KBr) : 3324, 3240 (NH-NH2), 1603 (C=O)
Spectre RMN 1H (CDCl3) : 3,82 (s, 3H, OCH3), 4,01 (s, 2H, NH2), 6,38 (s, 2H, (d, J2-3: 15,58 Hz, 1H, H2), 6,92 (d, J6'-5' : 6,79 Hz, 1H, H6'), 7,02 (d, J2'-6' : 1,91 Hz, 1H, H2'), 7,11 (d, J4'-5' : 7,79Hz, 1H, H4'), 7,14 (s, 1H, NH), 7,30 (m, 1H, H5'), 7,67 (d, J3-2 : 15,60 Hz, 1H, H3).

### Exemple 19 : (2E)-3-(3-hydroxyphényl)acrylohydrazide

On utilise le même mode opératoire que le procédé B de l'exemple 14, à partir de 3,07 g (0,01 mole) de N-acylbenzotriazole obtenu dans l'exemple 6, et de 0,6 g (0, 012 mole) d'hydrazine hydratée.

On obtient 0,7 g d'une poudre blanche
Point de fusion : 132°C
Spectre IR (KBr) : 3400, 3290, 3200 (OH, NH-NH2), 1682 (C=O)
Spectre RMN 1H (DMSO d6) : 4,07 (s, 2H, NH2), 6,58 (d, J2-3: 15,56 Hz, 1H, H2), 6, 79 (d, 1H, H4'), 6,85 (s, 1H, H2'), 6, 96 (d, 1H, H6'), 7,19 (q, 1H, H5'), 7,43 (d, J3-2: 15,60 Hz, 1H, H3), 8,50 (s, 1H, NH), 9,75 (s, 1H, OH).

### Exemple 21 : (2E)-3-(4-méthoxyphényl)acrylohydrazide

On utilise le même mode opératoire que le procédé B de l'exemple 14 à partir de 2,79g (0,01 mole) de N-acylbenzotriazole (composé de formule V) de l'exemple 8, et de 0,6 g (0,012 mole) d'hydrazine hydratée.

On obtient 1,75 g d'une poudre blanche.
Point de fusion : 140 °C
Spectre IR (KBr) : 3311, 3279 (NH NH2), 1655 (C=O)
Spectre RMN 1H (DMSO d6) : 3,76 (s, 3H, OCH3), 4,40 (s, 2H, NH2), 6,40 (d, J2-3: 15,60 Hz, 1H, H2), 6,96 (d, 2H, H2'6'), 7,39(d, J3-2 : 15,58 Hz, 1H, H3), 7,49 (d, 2H, H3'5'), 9,23 (s, 1H, NH)

### Exemple 22 : (2E)-3-(2-éthoxyphényl)acrylohydrazide

On utilise le même mode opératoire que le procédé B de l'exemple 14 à partir de 2,93g (0,01 mole) de N-acylbenzotriazole (composé de formule V) de l'exemple 9, et de 0,6 g (0,012 mole) d'hydrazine hydratée.

On obtient 1,35 g d'une huile jaune pâle.
Spectre IR (KBr) : 3248, 3074 (NH NH2), 1655 (C=O)
Spectre RMN 1H (CDCl3) : 1,24 (t, 3H, CH2-CH3), 4,08 (q, 2H, CH2-CH3), 4,15 (s, 2H, NH2), 6,60 (d, J2-3 : 16,58 Hz, 1H, H2), 6,90 (multiplet, 2H, H5'6'), 7,29 (dd, 1H, H4'), 7,43 (d, J3'-4' : 8,70 Hz, 1H, H3'), 7,50 (s, 1H, NH), 8,01 (d, J3-2 : 16,60 Hz, 1H, H3)

### Exemple 23 : (2E)-3-[3,4-diméthoxyphényl]acrylohydrazide

On suit le procédé A : on agite à 70°C pendant 1h30 5 g (0,0225 mole) de 3,4-diméthoxycinnamate de méthyle (composé de formule II) dans 45 ml d'hydrazine hydratée. Après refroidissement, la solution est concentrée sous vide, le résidu formé est agité pendant une heure dans 60 ml d'eau. Le précipité est essoré, lavé avec 20 ml d'acétonitrile et séché.

On obtient 2,65 g d'une poudre blanche.
Point de fusion : 196 °C
Spectre IR (KBr) : 3322,3232 (NH), 1651 (C=O)
Spectre RMN 1H (DMSO d6) : 3,77 (s, 6H, (OCH3)2), 4,38 (s, 2H, NH2), 6,43 (d, J2-3: 15,58 Hz, 1H, H2), 6,97 (d, J5'-6' : 7,8 Hz, 1H, H5'), 7,08 (d, J6'-5' : 7,8 Hz, 1H, H6'), 7,12 (s, 1H, H2'), 7,38 (d, J3-2 : 15,60 Hz, 1H, H3), 9,20 (s, 1H, NH)

### Exemple 24 : (2E)-3-(2,5-diméthoxyphényl)acrylohydrazide

On utilise le même mode opératoire que le procédé B de l'exemple 14 à partir de 3,09 g (0,01 mole) de N-acylbenzotriazole (composé de formule V) de l'exemple 10, et de 0,6 g (0,012 mole) d'hydrazine hydratée.

On obtient 1,35 g d'une poudre jaune clair.
Point de fusion : 136 °C
Spectre IR (KBr) : 3299, 3204 (NH NH2), 1686 (C=O)
Spectre RMN 1H (CDC13) : 3,76 (s, 3H, OCH3), 3,81 (s, 3H, OCH3), 4,10 (s, 2H, NH2), 6,51 (d, J2-3 : 15,58 Hz, 1H, H3), 6,88 (multiplet, 2H, H4'3'), 7,00 (s, 1H, H6'), 7,29 (s, 1H, NH), 7,91 (d, J3-2 : 15,58 Hz, 1H, H3)

### Exemple 25 : (2E)-3-(3-hydroxy-4-méthoxyphényl)acrylohydrazide

On utilise le même mode opératoire que le procédé B de l'exemple 14 à partir de 3,37g (0,01 mole) de N-acylbenzotriazole de l'exemple 11, et de 0,6 g (0,012 mole) d'hydrazine hydratée.

On obtient 1,3 g d'une poudre couleur crème.
Point de fusion : 184 °C
Spectre IR (KBr) : 3340, 3335 (OH, NH NH2), 1652 (C=O)
Spectre RMN 1H (DMSO d6) : 3,77 (s, 3H, OCH3), 4,20 (s, 2H, NH2), 6,25 (d, J2-3: 15,2 Hz, 1H, H2), 6,92 (m, 3H, H2', H5, H'6'), 7,29 (d, J3-2 : 15,6 Hz, 1H, H3), 9,21, 9,18 (m, 2H, OH, NH)

### Exemple 26 : (2E)-3-(3-hydroxy-4-méthoxyphényl)acrylohydrazide

Selon le procédé A on agite à 70°C, pendant 1h30 5 g (0,024 mole) de férulate de méthyle (composé de formule II) dans 50 ml d'hydrazine hydratée. Après refroidissement, la solution est concentrée sous pression réduite, le résidu formé est agité pendant une heure dans 60 ml d'eau. Le précipité est essoré, lavé avec 20 ml d'acétonitrile et séché.

On obtient une poudre blanche.
Point de fusion : 158°C
Spectre IR (KBr) : 3415, 3308, 3244 (NH, OH), 1659 (C=O)
Spectre RMN 1H (DMSO d6) : 3,85 (s, 3H, OCH3), 4,15 (s, 2H, NH2), 6,13 (d, J2-3: 15,74 Hz, 1H, H2), 6,85 (d, J5'-6' : 7,99 Hz, 1H, H5'), 7,05 (d, J6'-5': 7,99 Hz, 1H, H6'), 7,16 (s, 1H, H2'), 7,42 (d, J3-2 : 15,72 Hz, 1H, H3), 9,23 (s, 1H, NH)

Le compose de l'exemple 26 peut également être préparé selon le procédé A, en suivant le même mode opératoire qu'indiqué ci-dessus, mais à partir de férulate d'éthyle.

On obtient une poudre rosée.
Point de fusion : 158 °C

Les spectres IR et RMN sont identiques à ceux obtenus pour le composé de l'exemple 6 préparé selon le procédé A.

Enfin, le composé de l'exemple 26 peut être obtenu selon le procédé B, en suivant le même mode opératoire que le procédé B de l'exemple 14 à partir de 3,37g (0,01 mole) du dérivé benzotriazole de l'exemple 7, et de 0,6 g (0,012 mole) d'hydrazine hydratée.

### Exemple 27 : (2E)-3-(3,4,5-triméthoxyphényl)acrylohydrazide

On utilise le même mode opératoire que le procédé B de l'exemple 14 à partir du N-acylbenzotriazole (composé de formule V) de l'exemple 12, et d'hydrazine hydratée.
On obtient 2,10g d'une poudre jaune
Point de fusion : 154 °C

### Exemple 28 : (2E)-3-(2,3,4-triméthoxyphényl)acrylohydrazide

On utilise le même mode opératoire que le procédé B de l'exemple 14 à partir de 3,39g (0,01 mole) de N-acylbenzotriazole (composé de formule V) de l'exemple 13, et de 0,6 g (0,012 mole) d'hydrazine hydratée.

On obtient 2,05 g d'une poudre blanche
Point de fusion : 148 °C
Spectre IR (KBr) : 3319, 3266 (NH NH2), 1650 (C=O)
Spectre RMN 1H (CDCl3) : 3,88 (s, 8H, OCH3), 4,09 (s, 2H, NH2), 6,43 (d, J2-3 : 15,58 Hz, 1H, H2), 6,38 (d, J6'-5': 8,79 Hz, 1H, H6'), 7,11 (s, 1H, NH), 7,22 (d, J5'-6' : 8,80 Hz, 1H, H5'), 7,83 (d, J3-2 : 15,62 Hz, 1H, H3)

### Préparation des dérivés guanidine de formule (I) et de leurs sels

### Exemple 29 : Nitrate d'imino{2-[(2E)-3-(4-hydroxy-3-méthoxyphényl)prop-2-ènoyl]hydrazino}méthanaminium, (FAG Nitrate)

On porte au reflux pendant 2h30 dans 45 ml d'acétonitrile 5g (0,024 mole) du dérivé hydrazide de l'exemple 26 en présence de 4,83g (0,024 mole) de nitrate de 3-5-diméthylpyrazole-1-carboxamidine. Après refroidissement, la solution est concentrée sous vide et le résidu est repris dans 50 ml d'éther éthylique, agité et filtré sur fritté.

On obtient 6,35 g d'une poudre blanche.
Point de fusion : 224 °C
Spectre IR (KBr) : 3394, 3343, 3281, 3155, 3020 (NH, NH3+, OH), 1694 (C=O)
Spectre RMN 1H (DMSO d6) : 3,79 (s, 3H, OCH3), 6,42 (d, J2-3 : 15,62 Hz, 1H, H2), 6,82 (d, J5'-6': 8,70 Hz, 1H, H5'), 7,06 (d, J6'-5' : 8,70 Hz, 1H, H6'), 7,15 (s, 1H, H2'), 7,40 (s, 3H, NH3+), 7,50 (d, J3-2 : 15,60 Hz, 1H, H3), 9,47, 9,60 (s, 2H, NH), 10,87 (s, H, OH)

### Exemple 30 : {2[(2E)-3-(4-hydroxy-3-méthoxyphényl)prop-2-ényl]hydrazino}(imino)méthaneamino (FAG)

On dissout à 80°C dans 50 ml d'eau 3 g (0,00958 mole) du composé de l'exemple 29.

Après refroidissement, la solution est acalinisée avec une solution saturée d'hydrogéno carbonate de potassium. Le précipité est essoré, lavé à l'eau et séché.

On obtient 2 g d'une poudre jaune.
Point de fusion : 198 °C
Spectre IR (KBr) : 3543, 3395, 3390, 3244 (OH, NH), 1700 (C=O)
Spectre RMN 1H (DMSO d6) : 3,77 (s, 3H, OCH3), 6,38 (d, J3-2: 15,74 Hz, 1H, H3), 6,76 (d, 1H, H5'), 6,92 (m, 3H, H6', NH), 6,91 (m, 3H, H6', NH), 7,14 (m, 6H, H2, OH, NH)
Spectre RMN 1H (D2O) : 3,73 (s, 3H, OCH3), 6,38(d, 1H, H2), 6,75 (d, 1H, H5'), 6,96 (d, 1H, H6'), 7,06 (s, 1H, H2'), 7,22 (d, J2-3 : 15,60 Hz, 1H, H3)

### Exemple 31 : chlorhydrate d'imino{2-[(2E)-3-(4-hydroxy-3-méthoxyphényl)prop-2-ènoyl]hydrazino}méthanaminium

On agite à 50°C dans 25 ml d'éthanol absolu 0,5 g (0,002 mole) du dérivé de l'exemple 30, puis on ajoute goutte à goutte 0,25 ml d'acide chlorhydrique concentré, puis en laisse à cette température 2 heures. Après refroidissement, le précipité est essoré, lavé avec 5 ml d'acétonitrile et 5 ml d'éther éthylique et on sèche.

On obtient 0,48g d'une poudre blanche.

### Exemple 32 : bromhydrate d'imino{2-[(2E)-3-(4-hydroxy-3-méthoxyphényl)prop-2-ènoyl]hydrazino}méthanaminium

On suit le protocole de l'exemple 29, mais en faisant réagir 0,5 g (0,002 mole) du composé de l'exemple 30 avec 0,20 ml d'acide bromhydrique en solution à 33 % dans l'acide acétique

On obtient 0,5 g d'une poudre couleur crème.
Pont de fusion : 186°C

### Exemple 33 : sulfate d'amino{2-[(2E)-3-(4-hydroxy-3-méthoxyphényl)prop-2-ènoyl]hydrazino}méthanaminium

On suit le protocole général de l'exemple 29, à partir de 0,5g (0,002 mole) du composé de l'exemple 30 et de 0,25 ml d'acide sulfurique.

On obtient 0,58 g d'une poudre jaune clair.
Point de fusion : 228°C

### Exemple 34 : méthane sulfonate d'imino{2-[(2E)-3-(4-hydroxy-3-méthoxyphényl)prop-2-ènoyl]hydrazino}méthanaminium

On suit le protocole général de l'exemple 29, à partir de 0,5g (0,002 mole) du composé de l'exemple 30 et de 0,192 g (0,002 mole) d'acide méthane sulfonique.

On obtient 0,51 g d'une poudre blanche.
Point de fusion : 190°C

### Exemple 35 : benzoate d'imino{2-[(2E)-3-(4-hydroxy-3-méthoxyphényl)prop-2-ènoyl]hydrazino}méthanaminium

On suit le protocole général de l'exemple 29, à partir de 0,5g (0,002 mole) du composé de l'exemple 30 et de 0,244 g (0,002 mole) d'acide benzoïque.

On obtient 0,60 g d'une poudre blanche.
Point de fusion : 204°C

### Exemple 36 : glucoronate d'imino{2-[(2E)-3-(4-hydroxy-3-méthoxyphényl)prop-2-ènoyl]hydrazino}méthanaminium

On dissout 0,0776g (0,004 mole) d'acide D-glucuronique dans 13 ml d'eau sous agitation et on ajoute 25 ml d'alcool éthylique. Après 10 minutes d'agitation, on additionne 1 g (0,004 mole) du composé de l'exemple 30 et on chauffe la solution à 60°C pendant 2h30. Après refroidissement, on concentre sous pression réduite et reprend le résidu dans 20 ml d'acétonitrile. Après une heure d'agitation, le précipité obtenu est essoré, lavé avec 15 ml d'éther éthylique et séché.

On obtient 0,9 g d'une poudre blanche.
Point de fusion : 190°C
Spectre IR (KBr) : 3342, 3300, 3100, 2970 (NH, OH, NH3+), 1674 (C=O)

Spectre RMN 1H (DMSO d6) : 3,12 (m, 2H, H acide glucuronique), 3,41 (m, 2H, H acide glucuronique), 3, 78 (s, 3H, OCH3), 4,31 (d, 1H, H acide glucuronique), 4,89 (m, 1H, OH), 6,44 (d, J2-3: 15,62 Hz, 1H, H2), 6,81 (d, J5'-6' : 7,79 Hz, 1H, H5') 7,03 (d, J6'-5' : 7,80, 1H, H6'), 7,45 (s, 1H, H2'), 7,47 (d, J3-2 : 15,62 Hz, 1H, H3), 8,04, 10,50 (m, OH, NH, NH3+)

### Exemple 38 : nitrate d'imino {2-[(2E)-3-(1,3-benzodioxol-5yl)prop-2-ènoyl]hydrazine}méthanaminium

On suit le mode opératoire de synthèse de l'exemple 29 à partir de 2,06 g (0,01 mole) du composé de formule III de l'exemple 15 et de 2,01 g (0,01 mole) de nitrate de 3,5-diméthylpyrazole-1-carboxamidine.

On obtient 2,30 g d'une poudre blanche.
Point de fusion : 214°C
Spectre IR (KBr) : 3350, 3186, 2928, 2850 (NH-NH, C NH NH3+), 1666 (C=O)
Spectre RMN 1H (DMSO d6) : 6,07 (s, 2H, OCH2O), 6,44 (d, J2-3 : 15,58 Hz, H, H2), 6,98 (d, J6'-5' : 8,7 Hz, 1H, H6'), 7,14 (d, J6'-5' : 8,70 Hz, 1H, H5'), 7,19 (s, 1H, H2'), 7,47 (m, 3H, NH3+), 9,46, 10,14 (m, 3H, NH)

### Exemple 39 : nitrate d'imino {2-[(2E)-3-(nitrophényl)prop-2-ènoyl]hydrazine}méthanaminium

On suit le mode opératoire de synthèse de l'exemple 29 à partir de 2,07 g (0,01 mole) du composé de formule III de l'exemple 16 et de 2,01 g (0,01 mole) de nitrate de 3,5-diméthylpyrazole-1-carboxamidine.

On obtient 2 g d'une poudre blanche.
Point de fusion : 210°C
Spectre IR (KBr) : 3345, 3306, 3177, 3027 (NH-NH, C NH NH3+), 1696 (C=O)
Spectre RMN 1H (DMSO d6) : 6, 78 (d, J2-3 : 15,58 Hz, 1H, H2), 7,58 (m, 3H, NH3+), 7,72 (m, 2H, H5', C H3), 8,13 (d, J6'-5' : 8,7 Hz, 1H, H6'), 8,18 (d, J4'-5' : 8,85 Hz, 1H, H4'), 8,44 (s, 1H, H2'), 9,46, 10,00 (m, 2H, NH)

### Exemple 40 : nitrate d'imino {2-[(2E)-3-(méthoxyphényl)prop-2-ènoyl]hydrazine}méthanaminium

On suit le mode opératoire de synthèse de l'exemple 29 à partir de 1,92 g (0,01 mole) du composé de formule III de l'exemple 17 et de 2,01 g (0,01 mole) de nitrate de 3,5-diméthylpyrazole-1-carboxamidine.

On obtient 1,40 g d'une poudre blanche.
Point de fusion : 180°C
Spectre IR (KBr) : 3437,3308, 3174, 3021, 2959 (NH-NH, C NH NH3+), 1690 (C=O)
Spectre RMN 1H (DMSO d6) : : 3,86 (s, 3H, OCH3), 6, 68 (d, J2-3 : 15,58 Hz, 1H, H2), 7,02 (m, J5'-4' : 7,79 Hz, J5'-6' : 6,83 Hz, 1H, H5'), 7,11 (d, J6'-5' : 7,79 Hz, 1H, H6'), 7,42 (d, J4'-3' : 8,79 Hz, J4'-5' : 7,79 Hz, 1H, H4'), 7,55 ((d, J3'-4' : 15,58 Hz, 1H, H3) 7,50 (m, 3H, NH3+), 7,78 (d, J3-2 : 15,58 Hz, 1H, H3), 10,04 (m, 2H, NH)

### Exemple 41 : nitrate d'imino {2-[(2E)-3-(3-méthoxyphényl)-2-prop-2-ènoyl]hydrazino}méthanaminium

On suit le mode opératoire de synthèse du dérivé de l'exemple 29, à partir de 1,92 g (0,01 mole) du composé de formule III de l'exemple 18 et de 2,01 g (0,01 mole) de nitrate de 3-5-diméthylpyrazole-1-carboxamidine.

On obtient 1,8 g d'une poudre blanche.
Point de fusion : 182°C
Spectre IR (KBr) : 3431, 3291, 3173, 3018 (NH NH C NH NH3+) 1693 (C=O)
Spectre RMN 1H (DMSO d6) : 3,77 (s, 3H, OCH3), 6,61 (d, J2-3 : 16,58 Hz, 1H, H2), 6,99 (q, J6'-5' : 5,83 Hz, J6'-2' : 2,91 Hz, 1H, H6'), 7,15 (d, J2'-6' : 2,91 Hz, 1H,H2'), 7,19 (d, J4'-5' : 7,83 Hz, 1H, H4'), 7,35 (dd, 1H, H5'), 7,56 (d, J3-2 : 18,58 Hz, 1H, H3), 7,60 (m,3H, NH3+), 10,01 (m, 3H, NH)

### Exemple 42 : nitrate d'imino {2-[(2E)-3-(3-hydroxyphényl)-2-prop-2-ènoyl]hydrazino}méthanaminium

On suit le mode opératoire de synthèse du dérivé de l'exemple 29, à partir de 1,78 g (0,01 mole) du composé de formule III de l'exemple 19 et de 2,01 g (0,01 mole) de nitrate de 3-5-diméthylpyrazole-1-carboxamidine.

On obtient 0,8 g d'une poudre blanche.
Point de fusion : 178°C
Spectre IR (KBr) : 3420, 3290, 3180, 3020 (OH, NH NH C NH NH3+) 1690 (C=O)
Spectre RMN 1H (DMSO d6) : 6,34 (d, J2-3 : 15,58 Hz, 1H, H2), 6,64 (m, 2H, H2', H4'), 6,99 (q, J6'-5' : 6,10 Hz, H2, 1H, H6'), 7,30 (d, J5'-4' : 7,80 Hz, J5'-6' : 6,15 Hz, 1H, H5'), 7,40 (m, 3H, NH3+), 7,47 (d, J3-2 : 15, 58 Hz, 1H, H3), 8,40 (m, 3H, NH), 9,71 (s, 1H, OH)

### Exemple 43 : nitrate d'imino {2-[(2E)-3-(2-éthoxyphényl)-2-prop-2-ènoyl]hydrazino}méthanaminium

On suit le mode opératoire de synthèse du dérivé de l'exemple 29, à partir de 2,06 g (0,01 mole) du composé de formule III de l'exemple 22 et de 2,01 g (0,01 mole) de nitrate de 3-5-diméthylpyrazole-1-carboxamidine.

On obtient 1,6 g d'une poudre blanche.
Point de fusion : 184°C
Spectre IR (KBr) : 3437, 3316, 3171, 2989 (NH NH C NH NH3+) 1691 (C=O)
Spectre RMN 1H (DMSO d6) : 1,39 (t, 3H, CH2CH3), 4,11 (q, 2H, CH2CH3), 6,68 (d, J2-3 : 16,54 Hz, 1H, H2), 6,99 (dd, J5'-6' : 8,79 Hz, J5' -4' : 6,79 Hz, 1H, H5'), 7,09 (d, J6'-5' : 8,79 Hz, 1H, H6'), 7,37 (d, d, J4'-5' : 6,79 Hz, J4'-3' : 8,79 Hz, 1H, H4'), 7,54 (d, J3'-4' : 8,79 Hz, 1H, H3'), 7,60 (m, 3H, NH3+), 9,76, 10,20 (m, 3H, NH)

### Exemple 44 : nitrate d'imino {2-[(2E)-3-(2,5-diméthoxyphényl)-2-prop-2-ènoyl]hydrazino}méthanaminium

On suit le mode opératoire de synthèse du dérivé de l'exemple 29, à partir de 2,22 g (0,01 mole) du composé de formule III de l'exemple 24 et de 2,01 g (0,01 mole) de nitrate de 3-5-diméthylpyrazole-1-carboxamidine.

On obtient 2 g d'une poudre blanche.
Point de fusion : 208°C
Spectre IR (KBr) : 3402, 3380, 3184, 2960 (NH NH C NH NH3+) 1663 (C=O)
Spectre RMN 1H (DMSO d6) : 3,73 (s, 3H, OCH3), 3,81 (s, 3H, OCH3), 6,69 (d, J2-3 : 15,58 Hz, 1H, H2), 6,99 (d,d, J4'-3' : 8,75 Hz, J4'-6' : 2,91 Hz, 1H, H4'), 7,05 (d, J3'-4' : 8,75 Hz, 1H, H3'), 7,08 (d, J6'-4' : 2,91 Hz, 1H, H6'), 7,35 (m, 3H, NH3+), 7,74 (d, J3-2 : 15,58 Hz, 1H, H3), 10,04 (m, 3H, NH)

### Exemple 45 : {2-[(2E)-3-(2-éthoxyphényl)prop-2-ènoyl]hydrazino}(imino) méthane amino

On suit le mode opératoire de synthèse de l'exemple 30, à partir du composé de formule I de l'exemple 43.

On obtient une poudre jaune.
Point de fusion : 208°C
Spectre RMN 1H (DMSO d6) : 1,39 (t, 3H, CH2CH3), 4,05 (q, 2H, CH2CH3), 6,53 (d, J2-3 : 16,58 Hz, 1H, H2), 6,79 (m, 5H, NH-NH-C NH NH3+), 6,92 (m, 2H, H Ar), 7,20 (t, 1H, H Ar), 7,42 (d, J3-2 : 16,50 Hz, 1H, H3), 7,49 (d, 1H, H Ar)

### Exemple 46 : {2-[(2E)-3-(2,5-diméthoxyphényl)prop-2-ènoyl]hydrazino}(imino) méthane amino

On suit le mode opératoire de synthèse de l'exemple 30, à partir du composé de formule I de l'exemple 44.

On obtient une poudre jaune blanche.
Point de fusion : 172°C
Spectre RMN 1H (DMSO d6) : 3,71 (s, 3H, OCH3), 3,76 (s, 3H, OCH3), 6,60 (d, J2-3 : 15,62 Hz, 1H, H2), 6,70 (m, 5H, NH-NH-C NH NH3+), 6,80, 6,82 (m, 2H, H Ar), 7,70 (s, 1H, H6'), 7,36 (d, J3-2 : 15,60 Hz, 1H, H3)

### Exemple 47 : chlorhydrate d'imino{2-[(2E)-3-(2-éthoxyphényl)-2-prop-2-ènoyl]hydrazino}méthanaminium

On suit le mode opératoire de synthèse de l'exemple 31, à partir du composé de formule I de l'exemple 45.
On obtient une poudre beige.
Point de fusion : 212°C
Spectre RMN 1H (DMSO d6) : 1,38 (t, 3H, CH2CH3), 4,12 (q, 2H, CH2CH3), 6,72 (d, J2-3 : 15,62 Hz, 1H, H2), 7,00, 7,06 (m, 2H, H Ar), 7,36 (t, 1H, H Ar) 7,52 (d, 1H, H Ar), 7,60 (m, 4H, C NH NH3+), 7,78 (d, J3-2 : 15,62 Hz, 1H, H3), 9,67 (s, 1H, NH), 10,37 (s, 1H, NH)

### Exemple 48 : chlorhydrate d'imino{2-[(2E)-3-(2,5-diméthoxyphényl)prop-2-ènoyl]hydrazino}méthanaminium

On suit le mode opératoire de synthèse de l'exemple 31, à partir du composé de formule I de l'exemple 46.
On obtient une poudre beige.
Point de fusion : 220°C
Spectre RMN 1H (DMSO d6) : 3,72 (s, 3H, OCH3), 3,80 (s, 3H, OCH3), 6,78 (d, J2-3 : 15,60 Hz, 1H, H2), 7,01 (m, 2H, H Ar), 7,07 (s, 1H, H6'), 7,64 (m, 4H, C NH NH3+), 7,72 (d, J3-2 : 15,60 Hz, 1H, H3), 10,05 (s, 2H, NH-NH)

On va maintenant donner des exemples de compositions cosmétiques comprenant les dérivés de l'invention, chaque composition ayant été réalisée avec 1%, 2% et 5% de substance active.

### Composition N°1 : Crème hydrophile H/E

| | % | Constituants (nomenclature INCI) | Catégorie |
|---|---|---|---|
| | 2,00 | ceteareth-6, stearyl alcohol | tensio-actif non ionique |
| | 2,00 | ceteareth-25 | tensio-actif non ionique |
| | 4,00 | cetearyl alcohol | alcool gras |
| | 10,00 | cetearyl octanoate | tensio-actif non ionique |
| | 3,00 | glyceryl stearate | émollient, émulsionnant |
| | 5,00 | petrolatum | conditionneur, épaississant, protecteur |
| | 0,20 | EDTA | chélatant |
| | 5,00 | propylene glycol | humectant, solvant |
| | q.s. | preservative | |
| | q.s.p .100,00 | aqua | |
| | 1,00 (2,00 et 5,00) | substance active : composé n°29 | |

Chauffer les phases A et B séparément à 80°C Ajouter et mélanger la phase aqueuse B à la phase grasse A et homogénéiser parfaitement. Refroidir le mélange à 40°C et ajouter la phase C contenant la substance active sous agitation, homogénéiser, et poursuivre l'agitation jusqu'au retour à température ambiante.

### Composition N°2 : Crème lipophile E/H + filtre minéral anti UV-A

| | % | Constituants (nomenclature INCI) | Catégorie |
|---|---|---|---|
| | 5,00 | beeswax or beeswax substitute | stabilisateur d'émulsion, conditionneur, épaississant |
| | 9,00 | paraffinum liquidum | émollient, protecteur |
| | 50,50 | petrolatum | cire minérale, conditionneur,épaississant , protecteur |
| | 0,50 | aluminium stearate | tensio-actif émulsionnant H/E |
| | 10,00 | hydrogenated castor oil | conditionneur, tensio-actif émulsionnant |
| | 5,00 | CI 77891 (titanium dioxide) | pigment blanc opacifiant |
| | 0,7 | magnésium sulfate | vecteur, support d'actif |
| | q.s.p.100 ,00 | aqua | |
| | 1,00 (2,00 et 5,00) | substance active : composé n°29 | |

Mélanger les composants de la phase huileuse A et chauffer à 60°C. Le sulfate de magnésium est dissout dans l'eau et la phase aqueuse B est chauffée à 60°C.

Mélanger les 2 phases et ajouter la phase C contenant la substance active sous agitation, homogénéiser, et poursuivre l'agitation jusqu'au retour à température ambiante.

### Composition N°3 : Gel hydrophile

| | % | Constituants (nomenclature INCI) | Catégorie |
|---|---|---|---|
| | q.s.p. 100 | aqua | |
| | 0, 2 | preservative | |
| | 1, 00 (2,00 et 5,00) | substance active : composé n°29 | |
| | 24 ,00 | aqua | |
| | 0, 1 | preservative | |
| | 0, 75 | carbomer | gélifiant de phase aqueuse |
| | q.s.p. pH=6,5 | sodium hydroxide (N) | ajusteur de pH |

Préparer, dans un premier temps, séparément les fractions A et B, puis réaliser, dans un deuxième temps, la mise en gel.

Préparation de la fraction A : dissoudre le conservateur dans l'eau distillée à 50°C, y disperser ensuite la substance active sous agitation turbine et laisser refroidir le mélange résultant sous agitation planétaire jusqu'à température ambiante.

Préparation de la fraction B : dissoudre le conservateur dans l'eau distillée à 50°C, y disperser ensuite le carbomer sous agitation turbine et laisser refroidir le mélange résultant sous agitation planétaire jusqu'à température ambiante.

Pour obtenir le gel final, introduire à température ambiante et sous agitation turbine les phases B et C dans la phase A et ensuite homogénéiser le mélange par agitation planétaire.

### Composition N°4 : Lotion

| % | Constituants (nomenclature INCI) | Catégorie |
|---|---|---|
| 0,60 | PEG-40-hydrogenated castor oil | conditionneur, tensio-actif émulsionnant |
| 10,00 | glycerin | humectant |
| 0,3 | preservative | |
| q.s.p .100,00 | aqua | |
| 1,00 (2,00 et 5,00) | substance active : composé n°29 | |

Dissoudre le conservateur dans l'eau et la glycérine à 30°C sous agitation, puis ajouter la substance active sous agitation, ajouter ensuite le mélange PEG-40-hydrogenated castor oil (huile de ricin hydrogénée) sous agitation et filtrer le mélange résultant.

### Composition N°5 : Pommade

| % | Constituants (nomenclature INCI) | Catégorie |
|---|---|---|
| 7,0 0 | petrolatum | cire minérale, conditionneur, épaississant, protecteur |
| 13, 00 | microcristalline wax | cire minérale, émollient, protecteur |
| q.s.p. 100,00 | paraffinum liquidum | émollient, protecteur |
| 1,0 0 | substance active : composé n°29 | |

Au bain-marie, faire fondre les cires minérales dans la paraffine liquide (paraffinum liquidum) ; quand les cires sont fondues, retirer du bain-marie et bien remuer le mélange jusqu'à ce qu'il commence à prendre, ensuite ajouter la substance active et bien mélanger.

### Composition N°6 : Pommade

| % | Constituants (nomenclature INCI) | Catégorie |
|---|---|---|
| 25, 00 | beeswax | stabilisateur d'émulsion, conditionneur, épaississant |
| q.s.p. 100,00 | mineral oil | conditionneur, émollient |
| 1,00 (2,00 et 5,00) | substance active : composé n°29 | |

Au bain-marie, faire fondre la cire blanche (beeswax) dans la paraffine liquide (paraffinum liquidum) ; quand la cire est fondue, retirer du bain-marie et bien remuer le mélange jusqu'à ce qu'il commence à prendre, ensuite ajouter la substance active et bien mélanger.

On a évalué les propriétés anti-âge d'un dérivé selon l'invention, en faisant une étude comparative de l'activité anti-glycation de plusieurs composés.

Les produits issus de la glycation ont été mis en évidence par l'observation de la morphologie générale des structures dermique et épidermique des explants traités ainsi que par l'immunomarquage de la fibrilline-1 qui est une protéine servant de composant structurel aux microfibrilles.

### Mode opératoire :

A partir d'un bras de femme de 32 ans, 21 explants (d'un diamètre d'environ 10 mm) ont été préparés et mis en survie en milieu spécifique BEM (BIO-EC's Explants Médium).

Les explants ont été répartis en 7 lots de 3 explants :

| Lot | Nb explants | Traitement | Prélèvement |
|---|---|---|---|
| T0 | 3 | Aucun | J0 |
| T | 3 | Aucun | J9 |
| R | 3 | Aminoguanidine à 0,15 % | J9 |
| P | 3 | Composé n° 29 à 1% | J9 |
| T MG | 3 | Méthylglyoxal | J9 |
| R MG | 3 | Aminoguanidine à 0,15 % + Méthylglyoxal | J9 |
| P MG | 3 | Composé n°29 à 1% + Méthylglyoxal | J9 |

L'aminoguanidine est un composé anti-glycation couramment utilisé dans l'art antérieur et constitue la référence dans cette étude.

Le méthylglyoxal est un agent de glycation.

### Mode opératoire

Le méthylglyoxal a été incorporé dans le milieu de survie à J3, J5 et J7.

Le composé n° 29 et la référence, l'aminoguanidine, ont été appliqués en topique à raison de 2 mg par explant à J0, J3, J5 et J7.

Les témoins (lots T0 et T) n'ont reçu aucun traitement.

A J0, les trois explants du lot T0 ont été prélevés et coupés en deux. Une moitié a été fixée au formol tamponné et l'autre moitié congelée à -80°C. A J9, 3 explants de chaque lot ont été prélevés et traités de la même manière.

Après 24 heures de fixation dans le formol tamponné, les prélèvements ont été déshydratés et imprégnés en paraffine à l'aide d'un automate de déshydratation Leica 1020. Ils ont été mis en bloc à l'aide d'une station d'enrobage Leica EG 1160. Des coupes de 5 µm ont été réalisées à l'aide d'un microtome type Minot Leica RM 2125 et collées sur des lames de verre histologiques Superfrost^{®}.

L'observation de la morphologie générale a été effectuée sur des coupes en paraffine après coloration au trichrome de Masson, variante de Goldner.

La fibrilline-1 a été marquée à l'aide d'un anti-corps monoclonal anti-fibrilline1 clone 11.C1.3, de chez Neo Markers (ref MS 231), fait sur souris au 1/200ème pendant une heure à température ambiante avec un système amplificateur Vectastain RTU Universal VECTOR avidine/biotine et révélé en FITC. Les noyaux ont été colorés à l'iodure de propidium. Le marquage a été quantifié par des mesures en analyse d'image avec le système Q-WIN LEICA.

### Résultats

A J0, la morphologie des explants du lot T0 témoin est telle que le stratum corneum est peu épais, assez compact, assez nettement kératinisé en surface et très nettement à sa base. L'épiderme présente 4 à 5 assises cellulaires avec une très bonne morphologie. Le relief de la jonction dermo-épidermique est très net. Le derme papillaire présente un collagène avec des fibres assez épaisses formant un réseau assez dense. Il est bien cellularisé.

A J9, sur les explants du lot non traité T, la morphologie générale est proche de celle observée à J0.

Quelque soit le produit appliqué sur les explants des lots traités, la morphologie générale est peu modifiée, avec une parakératose plus ou moins présente et une spongiose plus ou moins nette en basale.

Concernant la fibrilline-1, à J0, sur les explants du lot non traité T0, le marquage est net, très régulier sur des fibres oxytalanes assez longues et bien ramifiées le long de la jonction dermo-épidermique. Il est très modéré sur les fibres élastiques du réseau sous-jacent dans le derme papillaire.

A J9, sur les explants du lot non traité T, l'expression de la fibrilline-1 est légèrement plus forte que celle observée à J0 : le marquage est très net, régulier.

Sur le lot R, traité à l'aminoguanidine, l'expression de la fibrilline-1 est proche de celle observée sur le lot témoin non traité T.

Sur le lot traité P avec le dérivé guanidine de l'invention, l'expression de la fibrilline-1 est proche de celle observée sur le lot témoin non traité T.

Après incorporation du méthylglyoxal, sur le lot T MG, l'expression de la fibrilline-1 est modérément diminuée, et assez irrégulière, par rapport à celle du lot témoin T sans méthylglyoxal.

Sur le lot R MG traité avec l'aminoguanidine en présence de méthylglyoxal, l'expression de la fibrilline-1 est très nette et plus forte que celle observée sur le lot T MG témoin avec méthylglyoxal.

Sur le lot P MG traité avec le dérivé guanidine de l'invention en présence de méthylglyoxal, l'expression de la fibrilline-1 est très nette et plus forte que celle observée sur le lot T MG témoin avec méthylglyoxal.

De plus, les résultats d'analyse d'images sont rassemblés dans le tableau ci-dessous.

### Fibrilline-1 (% de surface sous la jonction dermo-épidermique)

| | J0 | | J9 sans méthylglyoxal | | J9 avec méthylglyoxal | |
|---|---|---|---|---|---|---|
| | Moyenne | Ecart type | Moyenne | Ecart type | Moyenne | Ecart type |
| Témoin | 15,0 | 2,8 | 23,7 | 5,4 | 18,2 | 3,4 |
| r | | | 28,3 | 7,2 | 21,0 | 5,3 |
| p | | | 21,53 | 5,09 | 27,7 | 4,6 |

Dans le lot témoin T MG, l'incorporation de méthylglyoxal dans le milieu de survie induit une diminution de 23% du pourcentage de surface occupée par la fibrilline-1 sous la jonction dermo-épidermique, par rapport au lot T témoin sans méthylglyoxal.

Dans le lot R MG traité à l'aminoguanidine, l'incorporation de méthylglyoxal dans le milieu de survie induit une diminution de 12% du pourcentage de surface occupée par la fibrilline-1 sous la jonction dermo-épidermique, par rapport au lot T témoin sans méthylglyoxal.

Dans le lot P MG traité avec le dérivé guanidine de l'invention, l'incorporation de méthylglyoxal dans le milieu de survie induit une augmentation de 17% du pourcentage de surface occupée par la fibrilline-1 sous la jonction dermo-épidermique, par rapport au lot T témoin sans méthylglyoxal.

Ainsi, le méthylglyoxal induit une diminution nette de l'expression de la fibrilline-1 dans le lot T non traité, le traitement à l'aminoguanidine permet de réduire cette diminution de 49 % tandis que le traitement avec le dérivé guanidine de l'invention inhibe totalement l'action du méthylglyoxal.

## Revendications

1. Dérivés guanidines en série cinnamique, de formule générale (I) suivante, : 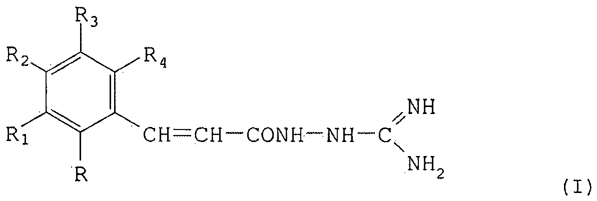 dans laquelle :
- R représente un atome d'hydrogène ou un groupe alkoxy en C1-C4,
- R1 représente un atome d'hydrogène, un groupe alkoxy en C1-C4, un groupe NO2 ou un groupe OH,
- R2 représente un atome d'hydrogène, un groupe alkoxy en C1-C4 ou un groupe OH,
- R1 et R2 peuvent également former ensemble un groupe OCH2O,
- R3 représente un atome d'hydrogène ou un groupe alkoxy en C1-C4,
- R, R1, R2 et R3 ne pouvant simultanément représenter un atome d'hydrogène, et
- R4 représente un atome d'hydrogène,
ainsi que leurs sels et leurs isomères.

2. Dérivés, isomères et sels selon la revendication 1, de formule (I) dans laquelle l'ensemble (R, R1, R2, R3, R4) est choisi dans le groupe constitué par (H, NO2, H, H, H), (alkoxy en C1-C4, H, H, H, H) et (H, alkoxy en C1-C4, OH, H, H).

3. Dérivés, isomères et sels selon la revendication 1, de formule (I) dans laquelle R1 et R2 forment ensemble un groupe OCH2O, et R, R3 et R4 représentent chacun un atome d'hydrogène.

4. Sel selon l'une des revendications 1 à 3, lequel est un sel d'addition d'un acide minéral ou organique.

5. Sel selon la revendication 4, qui est un sel d'addition de HCl, HBr ou H2SO4.

6. Sel selon la revendication 4, qui est un sel d'addition de l'acide méthanesulfonique, l'acide benzoïque, l'acide salicylique, l'acide lactique, l'acide citrique, l'acide malique D ou L, l'acide glucuronique D ou l'acide hyaluronique.

7. Procédé de préparation des dérivés, isomères et sels selon la revendication 1, **caractérisé en ce qu'**il comprend :
(i) la réaction du nitrate de 3,5-diméthylpyrazole-1-carboxamidine avec un composé de formule générale (III) suivante : 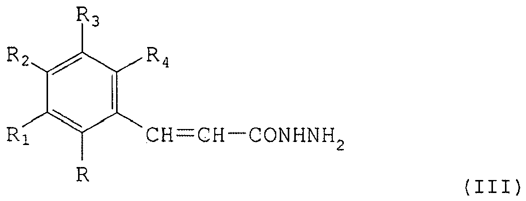 dans laquelle R, R1, R2, R3 et R4 ont la même signification que dans la revendication 1,
pour obtenir le nitrate d'un composé de formule (I),
(ii) l'alcalinisation du nitrate obtenu à l'étape (i), pour obtenir le composé de formule (I), et
(iii) la salification éventuelle du composé de formule (I) obtenu à l'étape (ii) par un acide minéral ou organique approprié.

8. Procédé selon la revendication 7, **caractérisé en ce qu'**il comprend la réaction de l'hydrazine avec un composé de formule générale (II) suivante : 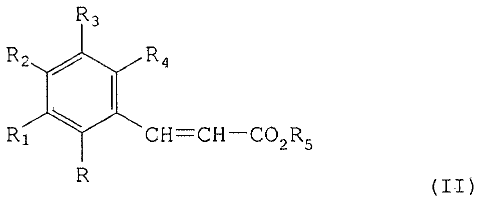 dans laquelle R, R1, R2, R3 et R4 ont la même signification que dans la revendication 1 et R5 représente un groupe alkyle en C1-C4,
pour obtenir le composé de formule (III).

9. Procédé selon la revendication 7, **caractérisé en ce qu'**il comprend :
(iv) la réaction du 1-(méthylsulfonyl)benzotriazole avec un composé de formule générale (IV) suivante : 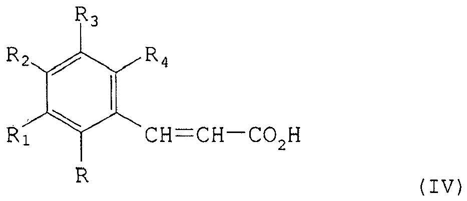 dans laquelle R, R1, R2, R3 et R4 ont la même signification que dans la revendication 1,
pour obtenir un composé de formule générale (V) suivante : 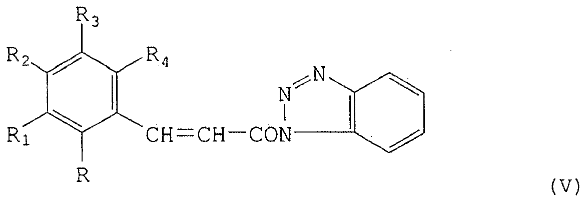 dans laquelle R, R1, R2, R3 et R4 ont la même signification que dans la revendication 1, et
(v) la réaction du composé de formule (V) obtenu à l'étape (iv) avec de l'hydrazine pour obtenir le composé de formule (III).

10. Utilisation des dérivés, sels et isomères selon l'une quelconque des revendications 1 à 6 pour la préparation d'une composition anti-glycation.

11. Utilisation des dérivés, sels et isomères selon la revendication 10, **caractérisée en ce que** ladite composition est une composition cosmétique.

12. Composition cosmétique comprenant au moins un dérivé, isomère ou sel selon l'une des revendications 1 à 6 et un véhicule acceptable sur le plan cosmétique.

## Patentansprüche

1. Guanidinderivate in der Zimtreihe, der folgenden allgemeinen Formel (I): 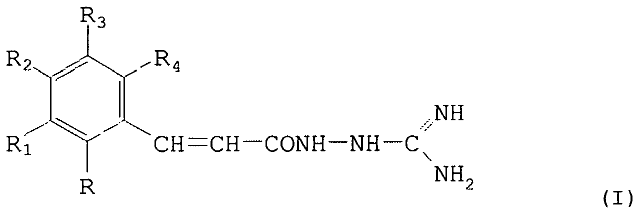 Wobei:
- R ein Wasserstoffatom oder eine Alkoxygruppe C1-4 darstellt,
- R1 ein Wasserstoffatom, eine Alkoxygruppe C1-C4, eine N02-Gruppe oder eine OH-Gruppe darstellt,
- R2 ein Wasserstoffatom, eine Alkoxygruppe C1-C4 oder eine OH-Gruppe darstellt,
- R1 und R2 gemeinsam auch eine OCH2O-Gruppe bilden können,
- R3 ein Wasserstoffatom oder eine Alkoxygruppe C1-C4 darstellt,
- R, R1, R2 und R3 nicht gleichzeitig ein Wasserstoffatom darstellen können, und
- R4 ein Wasserstoffatom darstellt,
sowie ihre Salze und Isomere.

2. Derivate, Isomere und Salze gemäß dem Patentanspruch 1, der Formel (I), wobei die Gesamtheit (R, R1, R2, R3, R4) aus der Gruppe ausgewählt wird, die sich aus (H, NO2, H, H, H), (Alkoxy C1-C4, H, H, H, H) und (H, Alkoxy C1-C4, OH, H, H)zusammensetzt.

3. Derivate, Isomere und Salze gemäß dem Patentanspruch 1, der Formel (I), wobei Ri und R2 gemeinsam eine OCH2O-Gruppe bilden,
und R, R3 und R4 jeweils ein Wasserstoffatom darstellen.

4. Salz gemäß einem der Patentansprüche 1 bis 3, welches ein Additionssalz einer Mineralsäure oder einer organischen Säure ist.

5. Salz gemäß dem Patentanspruch 4, das ein Additionssalz von HCl, HBr oder H2SO4 ist.

6. Salz gemäß dem Patentanspruch 4, das ein Additionssalz der Methansulfonsäure, der Benzoesäure, der Salicylsäure, der Milchsäure, der Zitronensäure, der Apfelsäure D oder L, der Glucuronsäure D oder der Hyaluronsäure ist.

7. Verfahren zur Herstellung von Derivaten, Isomeren und Salzen gemäß dem Patentanspruch 1, **dadurch gekennzeichnet, dass** es folgendes umfasst:
(i) die Reaktion des 3,5-Dimethylpyrazol-1-Carboxamidin-Nitrats mit einer Verbindung der allgemeinen Formel (III) wie folgt: 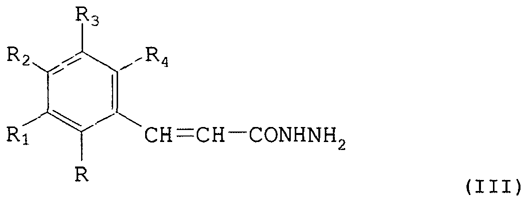 wobei R, R1, R2, R3 und R4 dieselbe Bedeutung wie in dem Patentanspruch 1 haben,
um das Nitrat einer Verbindung der allgemeinen Formel (I) zu erzielen,
(ii)die Alkalisierung des in Stufe (i) erzielten Nitrats, um die Verbindung in der Formel (I) zu erzielen, und
(iii) die mögliche Salzbildung der Verbindung der Formel (I),
die in Stufe (ii) durch eine geeignete Mineralsäure oder organische Säure erreicht wird.

8. Verfahren gemäß dem Patentanspruch 7, **dadurch gekennzeichnet, dass** es die Reaktion des Hydrazins mit einer Verbindung der allgemeinen Formel (II) wie folgt umfasst: 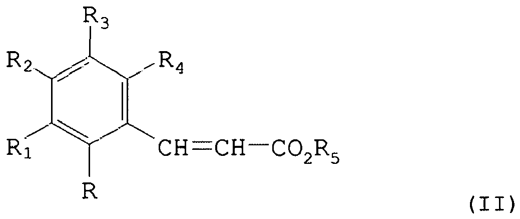 wobei R, R1, R2, R3 und R4 dieselbe Bedeutung wie in dem Patentanspruch 1 haben, und wobei R5 eine Alkylgruppe C1-C4 darstellt, um die Verbindung in der Formel (III) zu erzielen.

9. Verfahren gemäß dem Patentanspruch 7, **dadurch gekennzeichnet, dass** es folgendes umfasst:
iv) die Reaktion des 1-(Methylsulfonyl)Benzotriazols mit einer Verbindung der folgenden allgemeinen Formel (IV): 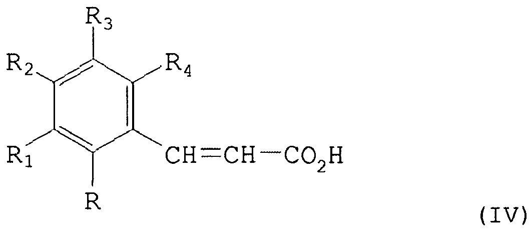 wobei R, R1, R2, R3 und R4 dieselbe Bedeutung haben wie in dem Patentanspruch 1, um eine Verbindung der folgenden allgemeinen Formel (V) zu erzielen: 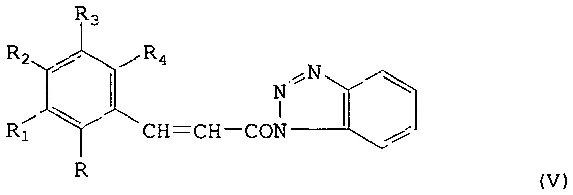 wobei R, R1, R2, R3 und R4 dieselbe Bedeutung haben wie in dem Patentanspruch 1, und
(v) die Reaktion der Verbindung der allgemeinen Formel (V), die in der Stufe (iv) mit Hydrazin erzielt wird, um die Verbindung der Formel (III) zu erzielen.

10. Verwendung der Derivate, Salze und Isomere gemäß einem beliebigen der Patentansprüche 1 bis 6 zur Herstellung einer Anti-Glykierungs-Zusammensetzung.

11. Verwendung der Derivate, Salze und Isomere gemäß dem Patentanspruch 10, die **dadurch gekennzeichnet ist, dass** die genannte Zusammensetzung eine kosmetische Zusammensetzung ist.

12. Kosmetische Zusammensetzung, die mindestens ein Derivat, Isomer oder Salz gemäß einem der Patentansprüche 1 bis 6 umfasst und einen kosmetisch annehmbaren Träger umfasst.

## Claims

1. Guanidine derivatives in the cinnamic acid series, of general formula (I) below: 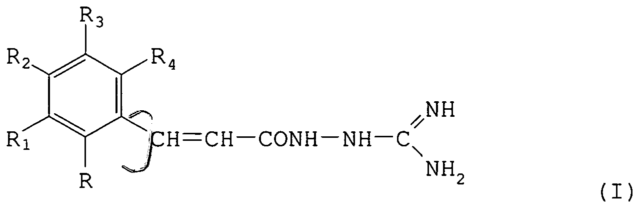 in which:
- R represents a hydrogen atom or a C1-C4 alkoxy group,
- R1 represents a hydrogen atom, a C1-C4 alkoxy group, a group NO2 or a group OH,
- R2 represents a hydrogen atom, a C1-C4 alkoxy group or a group OH,
- R1 and R2 may also together form a group OCH2O,
- R3 represents a hydrogen atom or a C1-C4 alkoxy group, and
- R, R1, R2 and R3 cannot simultaneously represent a hydrogen atom, and
- R4 represents a hydrogen atom,
and also salts thereof and isomers thereof.

2. Derivatives, isomers and salts according to Claim 1, of formula (I) in which the set (R, R1, R2, R3, R4) is chosen from the group consisting of (H, NO2, H, H, H), (C1-C4 alkoxy, H, H, H, H) and (H, C1-C4 alkoxy, OH, H, H).

3. Derivatives, isomers and salts according to Claim 1, of formula (I) in which R1 and R2 together form a group OCH2O, and R, R3 and R4 each represent a hydrogen atom.

4. Salt according to any one of Claims 1 to 3, which is an addition salt of a mineral or organic acid.

5. Salt according to Claim 4, which is an addition salt of HCl, HBr or H2SO4.

6. Salt according to Claim 4, which is an addition salt of methanesulfonic acid, benzoic acid, salicylic acid, lactic acid, citric acid, D or L malic acid, D glucuronic acid or hyaluronic acid.

7. Process for preparing the derivatives, isomers and salts according to Claim 1, **characterized in that** it comprises:
(i) the reaction of 3,5-dimethylpyrazole-1-carboxamidine nitrate with a compound of general formula (III) below: 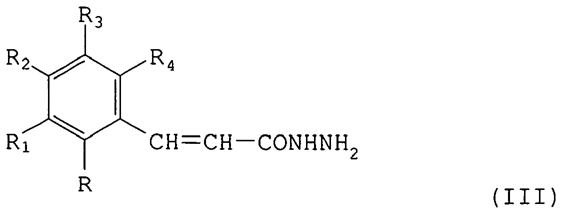 in which R, R1, R2, R3 and R4 have the same meaning as in Claim 1,
to obtain the nitrate of a compound of formula (I),
(ii) the basification of the nitrate obtained in step (i), to obtain the compound of formula (I), and
(iii) the optional salification of the compound of formula (I) obtained in step (ii) with a suitable mineral or organic acid.

8. Process according to Claim 7, **characterized in that** it comprises the reaction of hydrazine with a compound of general formula (II) below: 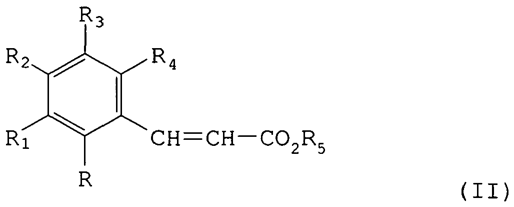 in which R, R1, R2, R3 and R4 have the same meaning as in Claim 1 and R5 represents a C1-C4 alkyl group,
to obtain the compound of formula (III).

9. Process according to Claim 7, **characterized in that** it comprises:
(iv) the reaction of 1-(methylsulfonyl)benzotriazole with a compound of general formula (IV) below: 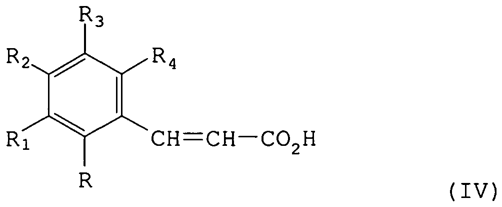 in which R, R1, R2, R3 and R4 have the same meaning as in Claim 1,
to obtain a compound of general formula (V) below: 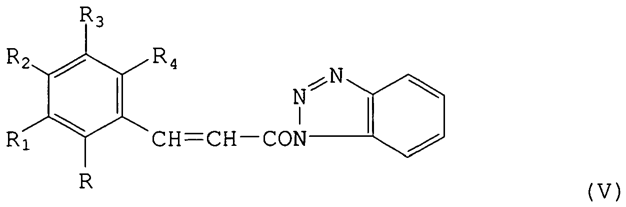 in which R, R1, R2, R3 and R4 have the same meaning as in Claim 1, and
(v) the reaction of the compound of formula (V) obtained in step (iv) with hydrazine to obtain the compound of formula (III).

10. Use of the derivatives, salts and isomers according to any one of Claims 1 to 6, for the preparation of an anti-glycation composition.

11. Use of the derivatives, salts and isomers according to Claim 10, **characterized in that** said composition is a cosmetic composition.

12. Cosmetic composition comprising at least one derivative, isomer or salt according to any one of Claims 1 to 6 and a cosmetically acceptable vehicle.
